# EUROPEAN PATENT SPECIFICATION

(11) **EP 0 970 131 B1**
(45) Date of publication and mention of the grant of the patent: **02.01.2004**
(21) Application number: 98912633.9
(22) Date of filing: 25.03.1998
(51) Int. Cl.: C08B 37/08, A61K 9/127

(54) **PARTICULATE DRUG CARRIERS**
TEILCHENFÖRMIGE ARZNEIMITTELTRÄGER
VECTEURS PARTICULAIRES DE MEDICAMENTS

(30) Priority: 25.03.1997 GB 9706195
(43) Date of publication of application: 12.01.2000
(73) Proprietor: UNIVERSITY OF STRATHCLYDE, Glasgow G1 1XQ (GB)
(72) Inventor: UCHEGBU, Ijeoma, Florence, Glasgow G61 3DT (GB)
(74) Representative: MacDougall, Donald Carmichael
(86) International application number: PCT/GB1998/000903
(87) International publication number: WO 1998/042755

(56) References cited:
- EP-A- 0 323 627
- EP-A- 0 773 229
- US-A- 4 904 772
- PATENT ABSTRACTS OF JAPAN vol. 18, no. 601 (C-1274), 16 November 1994 & JP 06 227965 A (SHIN ETSU CHEM CO LTD), 16 August 1994, & DATABASE WPI Week 9437 Derwent Publications Ltd., London, GB; AN 299679
- PATENT ABSTRACTS OF JAPAN vol. 11, no. 259 (C-441), 21 August 1987 & JP 62 059215 A (IHARA CHEM IND CO ), 14 March 1987,

## Description

### Field of the invention

This invention relates to particulate drug carriers formed from polysaccharide derivatives. A polysaccharide bearing at least one non-ionic hydrophilic group attached to the individual monosaccharide units is hydrophobised to form a derivative bearing at least one long chain alkyl residue. Particle formation is then induced in the presence of cholesterol. The particles are suited for entrapment or conjugation of pharmaceutically active ingredients.

### Backgroud to the invention

Chitosan (N-deacetylated chitin) has been investigated as a drug delivery agent when fabricated into cross-linked microspheres (Thanoo et al, J. Pharma. Pharmacol., 1992, 44, 283-286) and as a coating for liposomes (Henriksen et al, Int. J. Pharm., 1994, 101, 227-236). Chitosan solutions have also been used as penetration enhancers (Aspden et al, Eur. J. Pharm. Sci., 1996, 4, 23-32).

Yoshioka et al (Biosci. Biotech. Biochem., 1993, 57, 1053-1057) have shown that the precipitation (on standing) of a liposome suspension prepared from hydrogenated egg yolk lecithin could be prevented by treating the suspension with an aqueous solution of sulphated N-myristoyl chitosan (S-M-Chitosan). This result is explained by the surface of the liposomes being coated with S-M-chitosan, the ionisation of the sulphate group leading to a negative charge on the liposomes. This in turn causes repulsion between the liposome particles and prevents precipitation. The coating process was not found to destroy the lipid bilayer.

More recently, the same group (in Biosci. Biotech. Biochem., 1995, 59, 1901-1904) have examined the properties of aqueous solutions of chitosan derivatives, namely sulphated N-acyl chitosan (S-Cₙ-chitosan) having varying lengths of alkyl chain. S-Cₙ-chitosans from C₂ to C₁₄ dissolved completely in water to form transparent solutions. S-C₁₆-chitosan was also prepared but its properties were not examined since the aqueous mixture formed was not transparent and it was believed that an aggregate such as a liquid crystal had been formed. The solubilising capacity of the aqueous S-Cₙ-chitosan solutions towards a hydrophobic substance (azobenzene) was examined and it was found that solubility increased sharply with increasing carbon number above C₁₀. It was concluded by the authors that the long alkyl chains were aggregated to form micelles able to dissolve the azobenzene molecules. The micelles formed are described as "polymer micelles", although the type of micelle formed was not ascertained. The "polymer micelles" are suggested for use as drug carriers.

Sunamoto et al (Chem. Lett., 1991, 1263-1266) have reported that palmitoyl- or cholesterol-substituted derivatives of polysaccharides such as pullulan, amylopectin and dextran form self-aggregates in aqueous solution. A palmitoyl pullulan derivative substituted to 5.4 palmitoyl groups per 100 glucose units is reported, together with cholesterol-substituted pullulans having varying degrees of substitution. Investigation of the interaction between the pullulan derivatives and a fluorescent probe showed that the pullulan derivatives formed polymer self-aggregates above a critical concentration. The driving force for the aggregation is ascribed to a hydrophobic interaction between hydrophobic moieties and it is noted that the palmitoyl group was less effective for forming the self-aggregates. The aggregates are described as having the capacity to encapsulate various substances by hydrophobic interaction, for example drugs, proteins and nucleic acids.

The same group also reports (in Macromolecules, 1993, 26, 3062-3068) on the synthesis and solution properties of a non-ionic cholesterol-modified pullulan derivative (CHP) in water. In this work, pullulan was substituted by 1.6 cholesterol groups per 100 anhydroglucoside units. The authors state that the CHP self-aggregates formed relatively monodispersive particles and it is suggested that one CHP self-aggregate consists of approximately 13 CHP molecules. Experimental data suggests that the hydrophobic core of the CHP aggregates is completely and stably covered by the hydrophilic shell of the polysaccharide skeleton, forming colloidally stable nanoparticles above the critical concentration. The binding of various fluorescent probes was investigated and shown to increase with an increase in hydrophobicity of the probe. It is therefore concluded that the main driving force for complexation is a hydrophobic interaction.

These researches have further described (in Chem. Lett., 1995, 707-708) the complexation of the hydrogel nanoparticle formed by self-assembly of CHP with 5-10 insulin monomers in water. The number of complexed insulin molecules increased with an increase in the substitution degree of the cholesterol group of CHP. The insulin is stated as being complexed deeply inside the amphiphilic hydrogel matrix of the nanoparticle in which the hydrophobic microdomain of the associating cholesterol forms non-covalent crosslinks of gel structure. The number of crosslinks of one nanoparticle increases with an increase in the number of substitution degree of cholesterol, leading to an increase in the binding site for insulin.

Finally, the same group reports (in J. Am. Chem. Soc., 1996, 118, 6110-6115) a study of the complexation between CHP self-aggregate and bovine serum albumin (BSA). In all cases, approximately one BSA molecule was complexed by one nanoparticle of CHP self-aggregate, irrespective of the structure of the CHP self-aggregate. Unfolding of BSA by thermal means or by a denaturant such as urea was largely suppressed on complexation. This stabilisation of BSA upon complexation is ascribed to the formation of multiple non-covalent interactions between BSA and the hydrogel of CHP self-aggregate.

### Summary of the invention

According to the present invention, there is provided a pharmaceutical composition comprising particles, said particles comprising linear polysaccharide derivatives bearing at least one non-ionic hydrophilic group and at least one hydrophobic group per molecule and a pharmaceutically acceptable carrier wherein said hydrophilic group is attached to the individual monosaccharide units of said polysaccharide molecule and said hydrophobic group is attached to a monosaccharide unit of said polysaccharide molecule, and wherein said hydrophobic group comprises a C₁₂₋₂₄ alkyl, alkenyl, alkynyl or acyl residue.

The non-ionic hydrophilic group is preferably a group of the formula R¹, wherein R¹ is selected from mono- and oligo-hydroxy C₁₋₆ alkyl, mono- and oligo-hydroxy substituted C₂₋₆ acyl, C₁₋₂ alkoxy alkyl optionally having one or more hydroxy groups substituted on the alkoxy or alkylene groups, oligo- or poly-(oxa C₁₋₃ alkylene) preferably polyoxyethylene comprising up to about 120 ethylene oxide units (i.e. up to a molecular weight of 5000), and C₁₋₄ alkyl (oligo- or poly-oxa C₁₋₃ alkylene) optionally hydroxy substituted preferably oligo- or polyglycerol ethers such as those described in GB-A-1,529,625, for example containing up to 10 glycerol units; and wherein R¹ is joined via an ether linkage to a saccharide unit of the polysaccharide. It is to be understood herein that the term acyl includes alkenoyl and alkynoyl groups as well as alkanoyl groups.

The requirement that the hydrophilic group is non-ionic is an important feature since a charged ionic group such as sulphate would repel anionic DNA which, in one embodiment, is associated with the particles as a means for gene delivery or vaccination.

The polysaccharide derivative is preferably a derivative of chitosan, pullulan or dextran and most preferably comprises 1,4-linked saccharide units. Normally, substitution by the non-ionic hydrophilic moiety occurs at the C6 position of a saccharide unit.

The hydrophobic group is preferably joined to a saccharide unit by an amide, ester, ether or amine linkage, most preferably by an amide linkage. In a further preferred embodiment, this group is substituted at the C2 position in a 1,4-linked saccharide unit.

The compound has a degree of substitution by non-ionic hydrophilic groups in the range 0.1-1.5, preferably greater than 0.9 and most preferably 1 per saccharide unit.

The ratio of hydrophilic:hydrophobic groups in the compounds of this invention is in the range 100:1 to 1:2, preferably between 10:1 and 2:1 more preferably 5:1 and 2:1. Compounds having a degree of hydrophobic substitution of 0.5 or above per hydrophilic group are found to be difficult to disperse due to the high hydrophobic burden. Consequently, compounds having a degree of substitution of 0.25 or less are preferred.

A preferred range of compounds according to the present invention are the N-substituted derivatives of poly-amino glycans most preferably N-acyl glycol chitosans, especially N-palmitoyl glycol chitosan (poly[β(1→4)-2-deoxy-2-hexadecanamido-6-0-(2-hydroxyethyl)-D-glucopyranose]. In this case, the presence of free amino groups is advantageous from a point of view of permitting complexing with anionic DNA. In addition, such groups could be used for the conjugation of drug molecules.

The present invention further provides a composition comprising particles formed from a compound having the formula: 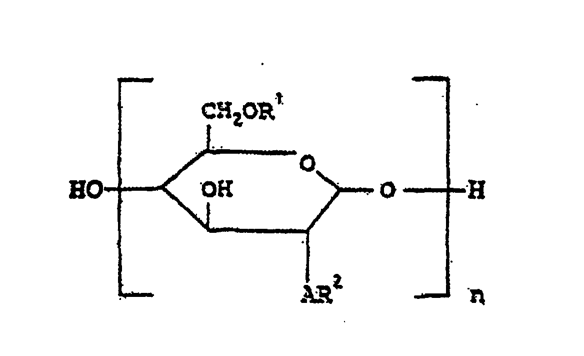 wherein each R¹ is selected from hydrogen, mono- and oligo-hydroxy C₁₋₆ alkyl, mono- and oligo-hydroxy substituted C₂₋₆ acyl, C₁₋₂ alkoxy alkyl optionally having one or more hydroxy groups substituted on the alkoxy or alkylene groups, oligo-or poly-(oxa C₁₋₃ alkylene) such as polyoxyethylene comprising up to about 120 ethylene oxide units and C₁₋₄ alkyl (oligo- or poly-oxa C₁₋₃ alkylene) optionally hydroxy substituted such as polyglycerol ethers, for example containing up to 10 glycerol units, provided that at least one of the groups R¹ is other than hydrogen;
A is -NH-, or -O-;
each R² is selected from hydrogen, C₁₂₋₂₄ alkyl, alkanoyl,-alkenyl, alkenoyl, -alkynyl or alkynoyl, provided that at least one of the groups R² is other than hydrogen; and
n is 5-2000.

Preferably, the group R¹ has the formula -CH₂CH₂OH or-CH₂CH(OH)CH₂OH, R² is C₁₆₋₁₈ acyl and A is -NH-.

In a further aspect, the present invention also provides a polysaccharide derivative bearing at least one non-ionic hydrophilic group and at least one hydrophobic group per polysaccharide molecule, wherein said hydrophilic group is substituted at the C6 position of a monosaccharide unit of said polysaccharide molecule via an amide linkage or amine linkage, and said hydrophobic group is substituted at the C2 position of a monosaccharide unit of said polysaccharide molecule via an amide linkage or amine linkage, and wherein said hydrophobic group comprises a C₁₂₋₂₄ alkyl, alkenyl, alkynyl or acyl residue.

In a yet further aspect, the present invention provides a polysaccharide derivative bearing at least one non-ionic hydrophilic group and at least one hydrophobic group per polysaccharide molecule for use in therapy, wherein said hydrophilic group is attached to a monosaccharide unit of said polysaccharide molecule and said hydrophobic group is attached to a monosaccharide unit of said polysaccharide molecule, and wherein said hydrophobic group comprises a C₁₂₋₂₄ alkyl, alkenyl, alkynyl or acyl residue.

In a still further aspect, the present invention provides use of a polysaccharide derivative bearing at least one non-ionic hydrophilic group and at least one hydrophobic group per polysaccharide molecule for the manufacture of a medicament for use in therapy, wherein said hydrophilic group is attached to a monosaccharide unit of said polysaccharide molecule and said hydrophobic group is attached to a monosaccharide unit of said polysaccharide molecule, and wherein said hydrophobic group comprises a C₁₂₋₂₄ alkyl, alkenyl, alkynyl or acyl residue.

The compounds may be formed according to any of the standard techniques described in the prior art for the derivatisation of polysaccharides (see for example, the references by Yoshioka et al -op cit). The technique may involve derivatisation of a polysaccharide starting material by a hydrophilic group in a first step, followed by a second step comprising attachment of a hydrophobic group or vice-versa. Alternatively, commercially-available polysaccharide derivatives already possessing a hydrophilic group may be hydrophobised using standard techniques to form a compound according to this invention.

The compounds described are used in combination with cholesterol or a derivative thereof to form particles. In the absence of cholesterol, particle formation does not occur and the material precipitates. Consequently, the presence of cholesterol is required to promote self-assembly of the polysaccharide derivatives to form particles.

The particles are made by techniques similar to those used to form liposomes and niosomes, for instance by blending the compounds in an organic solvent and then contacting the dried mixture with an aqueous solution, optionally followed by a particle size reduction step.

The particles formed may be suspended in an aqueous vehicle or alternatively may be isolated in a dry state. The particles may optionally incorporate a steric stabilizer, for instance a non-ionic amphiphilic compound, preferably a poly-24-oxyethylene cholesteryl ether. The particles may be micro or nano-particulate, nano-particles being formed preferably in the presence of the steric stabilizer. In this case, the steric stabilizer is incorporated into the structure of the particle.

The particles preferably also comprise an associated pharmaceutically active ingredient. The active ingredient may be water soluble, in which case it will be associated with the hydrophilic regions of the particle, or water insoluble and consequently associated with the hydrophobic regions of the particle.

Such an ingredient is preferably physically entrapped within the particle but may also be held by covalent conjugation. The pharmaceutically active ingredient may be a peptide or protein therapeutic compound. A further preferred alternative for the pharmaceutically active compound is nucleic acid (eg. DNA), preferably in the form of a gene for gene therapy or gene vaccination.

These drug carriers may be used for the treatment of a human or animal by therapy, in particular for oral drug delivery of peptides or proteins or as gene delivery vectors. It is envisaged that this drug delivery system will also be useful when used via the intravenous, intramuscular, intraperitional or topical (inhalation, intranasal, application to the skin) routes.

The novel compounds according to this invention may also be used for the coating of pre-formed liposomes or niosomes for instance which are drug carriers suspended in an aqueous carrier.

The invention will now be further illustrated with reference to the following non-limiting examples, wherein the fluorescent aqueous marker 5(6)-carboxy fluoroscein (CF) is used as a model drug and with reference to the figures in which:

Figure 1 shows the stability of bleomycin GCP41 based vesicles after storage at 4°C (●, ■) and room temperature 16 - 25°C (○, □). ■, □ = % encapsulation, ●, ○ = mean size. Data points = mean ± s.d., n = 3;

Figure 2 shows the release of 5(6)-carboxyfluorescein from GCP41, cholesterol vesicles. Data points = mean of 3 determinations. Δ = palmitoyl glycol chitosan based vesicles (mean ± s.d., n = 6), ▲ = Span 60 vesicles (mean, n = 3), ◆ = 5(6)-carboxyfluorescein solution;

Figure 3 shows the biocompatibility of GCP41 based vesicles against 3 cell lines, ■ = A549, ● = A431, ▲ = A2780. Data points = mean ± s.d., n = 3; and

### Detailed description of the invention

### Example 1

### SYNTHESIS OF N-PALMITOYL GLYCOL CHITOSAN (poly[β(1→4)-2-deoxy-2-hexadecanamide-6-0-(2-hydroxyethyl)-D-glucopyranose]

### a) GCP41 (4:1 initial ratio of glycol chitosan: palmitoyl units)

200mg glycol chitosan (GC) and 150mg sodium bicarbonate was dissolved in 35mL water. 10mL absolute ethanol was added, followed by a drop-wise addition of a solution of 79mg palmitoyl N-hydroxysuccinimide ester dissolved in 60mL absolute ethanol. Addition of the palymitoyl N-hydroxysuccinimide ester was carried out with stirring over 30min. The reaction mixture was initially cloudy but turned clear after about 1h. The reaction mixture was left to stir for 72h. After this time, 100mL acetone was added with formation of a slight precipitate. This mixture was then evaporated to reduced volume under reduced pressure at 60°C. The resuting liquid was extracted with 3 volumes of ether and exhaustively dialysed against water for 24h. The dialyzed mixture was freeze dried to give a white fluffy cotton wool like substance.

### b) GCP21 (initial 2:1 ratio of glycol chitosan: palmitoyl units)

200mg glycol chitosan (GC) and 150mg sodium bicarbonate was dissolved in 35mL water. 10mL absolute ethanol was added, followed by a drop-wise addition of a solution of 150mg palmitoyl N-hydroxysuccinimide ester dissolved in 120mL absolute ethanol. Addition of the palmitoyl N-hydroxysuccinimide ester was carried out with stirring over 30min. The reaction mixture was initially cloudy but turned clear after about 6h. The reaction mixture was left to stir for 72h. After this time, 100mL acetone was added with formation of a slight precipitate. This mixture was then evaporated to reduced volume under reduced pressure at 60°C. The resulting liquid was extracted with 3 volumes of diethyl ether and exhaustively dialyzed against water for 24h. The dialyzed mixture was freeze dried to a white fluffy cotton wool like substance. This was washed with water and the sticky mass freeze dried to give a fluffy cotton wool like substance.

### c) Characterisation of GCP41

### ¹H NMR.

Glycol chitosan is moderately soluble in water (2mg mL⁻¹) and ¹H NMR (with integration) and H ¹- H ¹COSY experiments were carried out on glycol chitosan in (D₂O, Sigma Chemical Co., UK) and GCP41 in a CD₃OD/D₂O mixture using a Bruker AMZ 400MHz in order to assign the non-exchangeable coupled protons.

### FT-IT.

FT-IR was performed in potassium bromide discs on a Mattson Galaxy FT-IR.

The level of hydrophobic modification in GCP41 and the original level of acetylation in glycol chitosan were assessed by ¹H NMR (Vårum et al 1991, Yoshioka et al 1993). In this way the batch of glycol chitosan (Sigma Chemical Co, UK - 105H0111) that was used was found to be one third acetylated. Proton assignments,
δ0.86p.p.m = CH₃ (palmitoyl) δ1.25p.p.m = CH₂ (palmitoyl), δ1.89p.p.m = CH₂ (palmitoyl - shielded by carbonyl), δ2.13p.p.m = CH₃ (acetyl - GCP41), δ2.14p.p.m. = CH ₂ (adjacent to carbonyl protons),
δ1.99p.p.m = CH₃ (acetyl - glycol chitosan), δ2.71p.p.m = CH (C2 sugar proton - GCP41), δ2.64p.p.m = CH (C2 sugar proton - GCP41), δ3.31p.p.m = methanol protons, δ3.3 - 4.0p.p.m = non-exchangeable sugar protons, δ4.4p.p.m = water protons. The level of hydrophobic modification in GCP41 was assessed by using the ratio of non-exchangeable C2 protons to methyl protons (spectrum b) and was found to be 14.48 ± 2.88% (mean ± s.d., n = 3) with values lying between 11 and 16 mole %. The ratio of N-acetyl protons, C2 sugar protons, 9 additional sugar/glycol non-exchangeable protons remains at (∼1:1:10) in all three spectra.

GCP41 was insoluble yet dispersible in D₂O to give a cloudy liquid which remained without a sediment for at least 4 weeks. The ¹H NMR spectra of a fresh sample of this dispersion is devoid of signals for the fatty acid side chain protons. This suggests that palmitoyl glycol chitosan in water adopts an orientation in which the fatty acid side chains exist in hydrophobic domains separated from the hydrophilic part of the polymer. The acetyl group appears to be an integral part of the hydrophilic portion of the molecule in the modified polymer as signals for the acetyl groups are clearly seen in the GCP41 - D₂O spectra. Hence there was no co-operative association between the acetyl group and the hydrophobic side chains when palmitoyl glycol chitosan was dispersed in water. Freeze fracture electron microscopy did not reveal the existence of any discernible particulate matter in this cloudy liquid.

The GCP41 FT - IR spectrum revealed a sharpening of the amide peak at 1648 cm⁻¹. The starting material glycol chitosan contains a relatively smaller amide peak at 1653 cm⁻¹.

### Example 2

### PREPARATION AND CHARACTERIZATION OF GCP41 AND GCP21 MICRO- AND NANO-PARTICLES

### a) GCP21 - Cholesterol Particles

7.2mg cholesterol was dissolved in 10mL chloroform. To this solution was added 12.2mg GCP21. The organic solvent was removed under vacuum and the solid deposit dried under a stream of nitrogen. 2mL of aqueous CF (5mM) was added to this solid deposit and the mixture shaken for 1h at 70°C to form a homogenous dispersion of micro-particles.

0.1mL of this dispersion was then fractionated over a Sephadex G50 column (205 x 8mm) and the sample eluting in the void volume collected. This sample was sized in a Malvern Mastersizer. Assay for entrapped material was carried out by solubilizing the particles in isopropanol (0.1mL dispersion to 1mL isopropanol). CF was then assayed by f luorometry (exc.= 486nm, em.= 514nm).

### b) GCP21 - Cholesterol - Solulan C24 Particles

6.2mg cholesterol and 5.4mg Solulan C24 were dissolved in 10mL chloroform. To this solution was added 11mg GCP21. The organic solvent was removed under vacuum and the solid deposit dried under a stream of nitrogen. 2mL of aqueous CF (5mM) was added to this solid deposit and the mixture shaken for 1h at 70°C to form a homogenous dispersion of micro-particles.

Nano-particles were prepared by filtration of this dispersion (0.22µm).

0.1mL of these dispersions was then fractionated over a Sephadex G50 column (205 x 8mm) and the sample eluting in the void volume collected. This sample was sized in a Malvern Mastersizer or Autosizer depending on the particle size. Assay for entrapped material was carried out by solubilizing the particles in isopropanol (0.1mL dispersion to 1mL isopropanol). CF was then assayed by fluorometry (exc.= 486,em.+ 514nm).

### c) GCP41 - Cholesterol Particles

7.3mg cholesterol was dissolved in 10mL chloroform. To this solution was added 19.8mg GCP41. The organic solvent was removed under vacuum and the sold deposit dried under a stream of nitrogen. 2mL of aqueous CF (5mM) was added to this solid deposit and the mixture shaken for 1h at 70°C to form a homogenous dispersion of micro-particles.

0.1mL of this dispersion was then fractionated over a Sephadex G50 column (205 x 8mm) and the sample eluting in the void volume collected. This sample was sized in a Malvern Mastersizer. Assay for entrapped material was carried out by solubilizing the particles in isopropanol (0.1mL dispersion to 1mL isopropanol). CF was then assayed by fluorometry (exc.= 486nm, em.= 514nm).

### d) GCP41 - Cholesterol - Solulan C24 Particles

6.5mg cholesterol and 5.4mg Solulan C24 were dissolved in 10mL chloroform. To this solution was added 17.3mg GCP41. The organic solvent was removed under vacuum and the solid deposit dried under a stream of nitrogen. 2mL of aqueous CF (5mM) was added to this solid deposit and the mixture shaken for 1h at 70°C to form a homogenous dispersion of micro-particles.

Nano-particles were formed by filtration of this dispersion (0.22µm).

0.1mL of this dispersion was then fractionated over a Sephadex G50 column (205 x 8mm) and the sample eluting in the void volume collected. This sample was sized in a Malvern Mastersizer or Autosizer. Assay for entrapped material was carried out by solubilizing the particles in isopropanol (0.1mL dispersion to 1mL isopropanol). CF was then assayed by fluorometry (exc.= 486nm, em.= 514nm).

The sizes and encapsulation efficiencies are given in Table 1.

### Example 3

### PREPARATION OF BLEOMYCIN ENTRAPPED VESICLES

GCP41 vesicles were prepared by the sonication of GCP41 (8mg) and cholesterol (4mg, Sigma Chemical Co., UK) in water for 2 X 2 minutes with the instrument set at 20% of its maximum capacity. Bleomycin GCP41 vesicles were prepared by sonicating GCP41(8mg) and cholesterol (4mg) in 2mL ammonium sulphate (0.12M, Sigma Chemical Co., UK). Unentrapped ammonium sulphate was removed by ultracentrifugation (150,000g X 1h - MSE 75 superspeed). Vesicles were then incubated for 1h at 60°C with bleomycin (Lundbeck, UK) solution (2mL 6U mL⁻¹) and left to stand overnight at room temperature. Unentrapped bleomycin was also removed by ultracentrifugation (150,000g X 1h) and entrapment was measured by disrupting the vesicles in 10X volume isopropanol (Rathburn Chemical Co., UK) followed by ultraviolet absorption spectrophotometry at 254nm (Unicam UV-1).

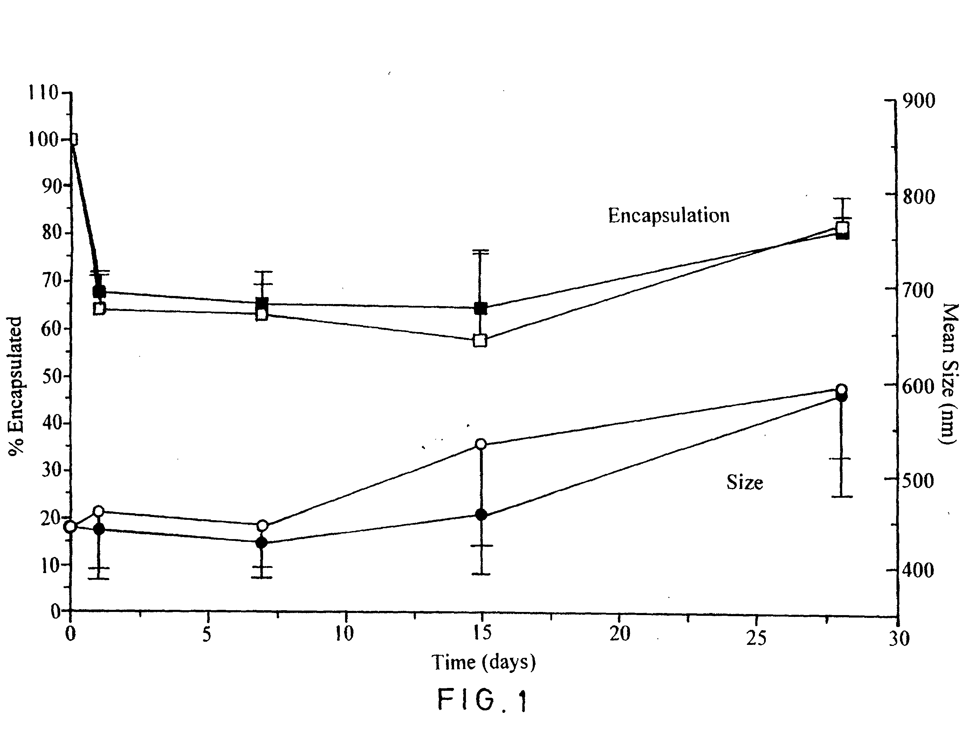

On storage at room temperature there was an initial loss of bleomycin although over 60% of the drug is retained within the vesicles (see Figure 1). Particle size is also seen to change very little. The stability data suggests that there is a loosely bound and a tightly bound fraction of bleomycin associating with GCP41 vesicles. The tightly bound fraction is presumed to be that fraction of bleomycin that traverses the membrane of the polymeric vesicle and actually accumulates within it in response to the ammonium sulphate gradient.

### Example 4

### 5(6)-CARBOXYFLUORESCEIN RELEASE FROM VESICLES

Vesicles were prepared as described in Example 3 from GCP41(16mg) and cholesterol (8mg) except that the hydrating solution was 4mL 5(6)-carboxyfluorescein (5.03mM, Sigma Chemical Co., UK). Sorbitan monostearate vesicles were prepared by hydrating sorbitan monostearate (24mg, Sigma Chemical Co., UK), cholesterol and poly-24-oxyethylene cholesteryl ether (16mg, D.F. Anstead, UK) in the presence of 4mL 5(6)-carboxyfluorescein (5.03mM). Unentrapped material was again removed by ultracentrifugation as described in Example 3. The release of 5(6)-carboxyfluorescein from GCP41 and sorbitan monostearate vesicles was monitored as follows. A 1:2 mixture of the vesicles and 2% w/w bile salts (sodium cholate and sodium deoxycholate, Sigma Chemical Co., UK) was placed in a 5cm piece of Visking tubing (Mw cut off 12,000 - 14,000) sealed at both ends. This mixture was dialysed against a 13-fold volume of the bile salts solution. 5(6)-carboxyfluorescein external to the dialysis tubing was monitored fluorimetrically (exc. 486, em. = 514nm, Perkin Elmer LS-5) at regular time intervals. A 1:2 mixture of 5(6)-carboxyfluorescein in phosphate buffered saline (PBS, pH = 7.4) (0.5mL) and 2%w/w bile salts (1mL) was included as a control.

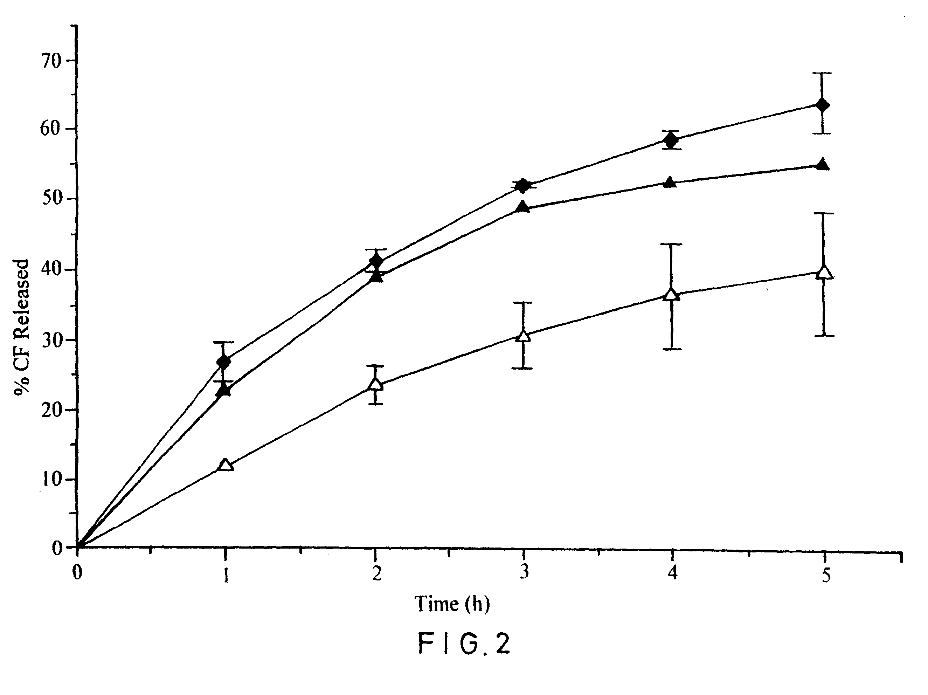

Using the release of the small molecular weight compound 5(6)-carboxyfluorescein (Mw = 387) as a marker for vesicle integrity, these polymeric vesicles are found to be more resistant to attack by detergents than vesicles prepared from the non-ionic surfactant sorbitan monostearate (see Figure 2). This is believed to be due to the difficulty the soluble bile salt surfactants have in inserting into a polymeric bilayer as opposed to the ease of insertion into a bilayer resulting from the self-assembly of monomers.

### Example 5

### BIOCOMPATIBILTY AND HAEMOCOMPATIBILITY STUDIES Biocompatibility studies

Cytotoxicity was evaluated by the IC50 value in a standard MTT based assay (Freshney et al Clulture of Animal Cells, 3rd edition, Wiley-Liss, New York, 1994). Depending on the growth rate, 0.5-2.0 X 10³ cells per well were seeded into 96 well plates and incubated for 24 h. Serial dilutions of the suspensions were added and incubated with the cells for 12 h. The suspensions were replaced with fresh medium and the cells were incubated with repeated feeding for 72 h. 50mg mL⁻¹ MTT [3-(4,5-dimethylthiazol-2-yl)-2,5-diphenyltetrazolium bromide, 50µL, Sigma Chemical Co., UK) was added to each well. After incubation for 4 h in the dark, the medium and MTT solution was removed and the cells were lysed in DMSO (200µL, Sigma Chemical Co., UK). Following the addition Sorensen's glycine buffer (25µL) the absorption was measured at 570nm.

### Haemocompatibility studies

Freshly drawn human blood was centrifuged (3,000g) to separate the red blood cells. These were washed in PBS (pH = 7.4) and weighed. 3g of the erythrocyte pellet was dispersed in 100mL PBS (pH = 7.4) and incubated for 5h with various concentrations of GCP41, cholesterol vesicles prepared as described above or DOTAP vesicles (Sigma Chemical Co., UK). Haemolysis was assessed by centrifugation (3,000g) to isolate the released haemoglobin, addition of 2X volume of isopropanol to the supernatant and the measurement of the absorbance (570nm).

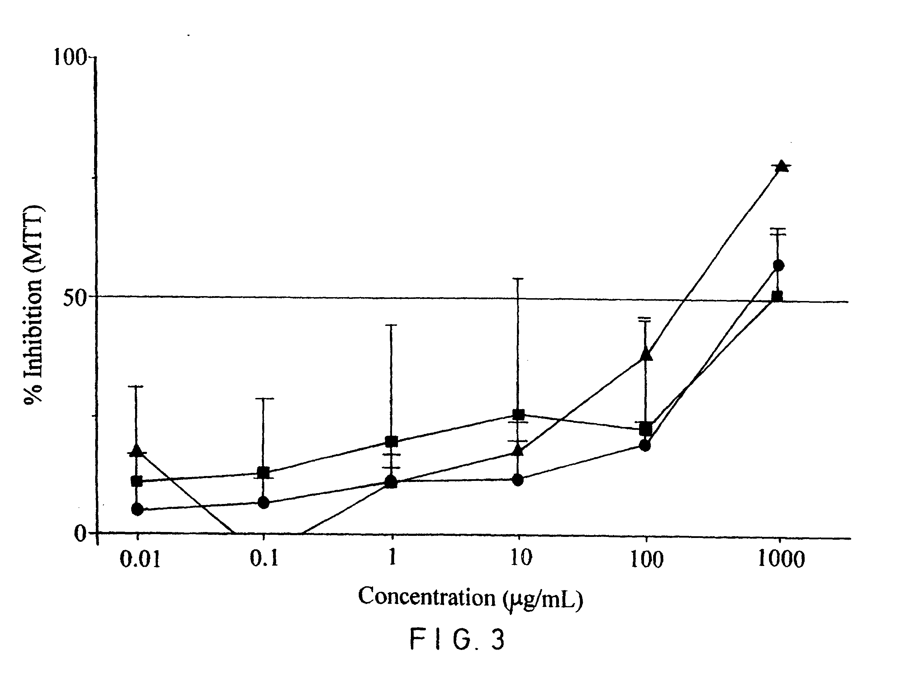

GCP41 vesicles were biocompatible with 3 human cell lines A2780 (ovarian cancer cell line), A549 (lung carcinoma) and A431 (epidermoid carcinoma) with no toxicity evident at concentrations of GCP41 below 150µg mL⁻¹ and IC50 values of 0.2, 1.0 and 1.0mg mL⁻¹ respectively (Figure 3). GPC41 vesicles showed good haemocompatibility with human erythrocytes and an ability to modulate the haemolytic activity of N-[1-(2,3-dioleoyloxy)propyl]-N,N,N-trimethylammonium methylsulphate (DOTAP) - the DNA transfection agent (Porteous et al, (1997) Gene Therapy 4, 210-218) (Table 2). These biocompatibility data are in good agreement with that reported for soluble glycol chitosan against the B16F10 cell line and rat erythrocytes (Carreno-Gomez and Duncan (1997) Int. J. Pharm. 148, 231-240.

### Example 6

### PREPARATION OF INSULIN AND LHRH ENTRAPPED VESICLES

GCP21 was prepared according to Example 1
a) Insulin. GCP21 vesicles were prepared by sonication of a mixture of GCP21 (8mg) and cholesterol (4mg) in 2mL water. GCP21 vesicles were loaded with insulin by either incubating 1ml of the vesicle dispersion with 1ml of insulin (160IU mL⁻¹) for 16h at room temperature or by the use of the dehydration-rehydration (DRV) method (Kirby C., Gregoriadis G (1984) Biotechnology 979-984) in which insulin vesicles mixtures, as described above, were lyophilised overnight and subsequently rehydrated to 2mL volume. The amount of insulin encapsulated was assessed by HPLC after separation of encapsulated insulin from the unencapsulated material by ultracentrifugation (150,000g) and disruption of the vesicles with isopropanol (1ml isopropanol to 1ml of the vesicle dispersion). Vesicles were also sized by photon correlation spectroscopy and the zeta potential of the dispersion measured.
b) Lutenizing hormone releasing hormone (LHRH) LHRH was loaded onto GCP21 vesicles by the use of ammonium sulphate gradients (Haran, G et al (1993) Biochym. Biophys. Acta 1151 , 201-205). Vesicles encapsulating ammonium sulphate were prepared by sonicating GP21 (8mg), cholesterol (4mg) mixtures in 2mL of a solution of ammonium sulphate (0.03M). Unentrapped ammonium sulphate was separated by ultracentrifugation (150,000g) and the pelleted ammonium sulphate vesicles were incubated with 2mL LHRH (2.5mg mL⁻¹). Unentrapped LHRH was also removed by ultracentrifugation (150,000g for one hour). These vesicles were also sized by photon correlation spectroscopy.

a) Insulin. Insulin GCP21 vesicles could be prepared by incubating pre-formed GCP21 vesicles with insulin at room temperature for approximately 16h (Table 3). No improvement in the level of insulin associated with the vesicles was observed with the DRV method. The zeta potential of the GCP21 vesicles increased from -5mV to +10mV on loading with insulin, indicating that the insulin associates with the surface of the vesicles to a certain extent.
b) LHRH. LHRH vesicles could also be prepared by the use of ammonium sulphate gradients (Table 4).

**Table 1:**

| Size and CF encapsulation efficient of GCP21 and GCP41 particles. | | |
|---|---|---|
| **PARTICLE** | **SIZE** | **% CF ENCAPSULATION** |
| GCP21/Cholesterol micro-particles | 34.6µm | 7.4% |
| GCP21/Cholesterol/Solulan C24 micro-particles | 30.7µm | 4.6% |
| GCP41/Cholesterol micro-particles | nd | 9.3% |
| GCP41/Cholesterol/Solulan C24 micro-particles | nd | 4.2% |
| GCP21/Cholesterol/Solulan C24 nano-particles | 325nm | 6.88% |
| GCP41/Cholesterol/Solulan C24 nano-particles | 333nm | 4.6% |

**Table 2:**

| The haemocompatibility of GCP41 vesicles | | |
|---|---|---|
| Formulation | Erythrocyte, polymer/DOTAP ratio | % Haemolysis (n=3)* |
| | | |
| DOTAP | 30 (erythrocyte, DOTAP ratio) | 101.4 ± 20.4 |
| DOTAP GCP41, cholesterol | 300 (erythrocyte, DOTAP ratio) | 71.0 ± 10.6 |
| (8:4) GCP41, cholesterol, | 30 (eythrocyte, GCP41 ratio) | 4.2 ± 1.6 |
| DOTAP (6:2:1) | 60 (erythrocyte, DOTAP ratio) | 10.7 ± 1.3 |
| GCP41, cholesterol, | | |
| DOTAP (6:2:1) | 600 (erythrocyte, DOTAP ratio) | 6.6 ± 1.8 |

| | | |
|---|---|---|
| *Haemocompatibility is expressed with respect to 100% haemolysis produced by an erythrocyte, triton X-100 weight ratio of 3:1 and 0% haemolysis produced by PBS (pH = 7.4). | | |

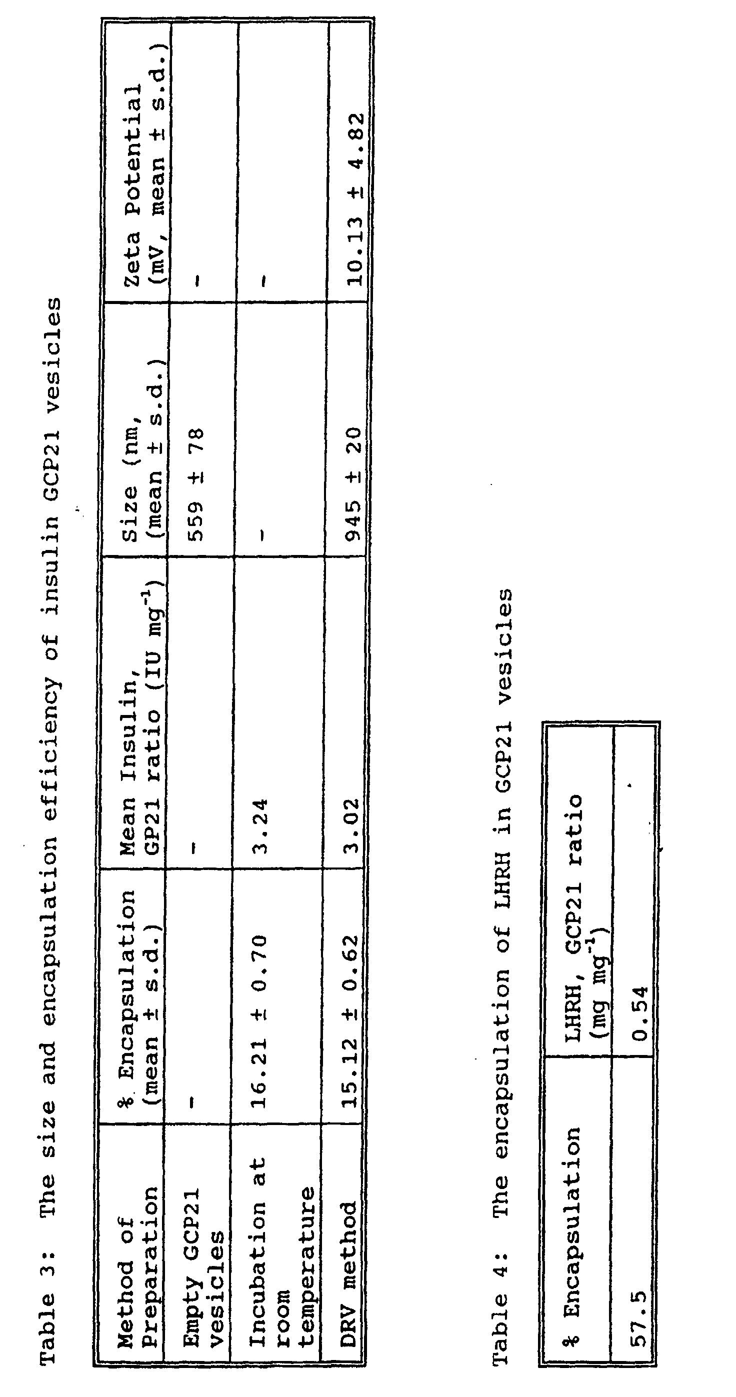

## Claims

1. A pharmaceutical composition comprising particles, said particles comprising linear polysaccharide derivatives, each polysaccharide derivative bearing at least one non-ionic hydrophilic group and at least one hydrophobic group per polysaccharide molecule and a pharmaceutically acceptable carrier, wherein said hydrophilic group is attached to a monosaccharide unit of said polysaccharide molecule and said hydrophobic group is attached to a monosaccharide unit of said polysaccharide molecule, and wherein said hydrophobic group comprises a C₁₂₋₂₄ alkyl, alkenyl, alkynyl or acyl residue.

2. A pharmaceutical composition according to claim 1 wherein the non-ionic hydrophilic group is a group R¹, where R¹ is selected from mono- and oligo-hydroxy C₁₋₆ alkyl, mono- and oligo-hydroxy substituted C₂₋₆ acyl, C₁₋₂ alkoxy alkyl, oligo- or poly- (oxa C₁₋₃ alkylene) and C₁₋₄ alkyl (oligo- or poly-oxa C₁₋₃ alkylene); and wherein R¹ is joined via an ether linkage to a monosaccharide unit of the polysaccharide.

3. A pharmaceutical composition according to claim 2 wherein said C₁₋₂ alkoxy alkyl has one or more hydroxy groups substituted on the alkoxy or alkylene groups.

4. A pharmaceutical composition according to claim 2 wherein said oligo- or poly- (oxa C₁₋₃ alkylene) is polyoxyethylene comprising up to 120 ethylene oxide units.

5. A pharmaceutical composition according to claim 2 wherein said C₁₋₄ alkyl (oligo- or poly-oxa C₁₋₃ alkylene) is hydroxy substituted.

6. A pharmaceutical composition according to claim 5 wherein said hydroxy substituted C₁₋₄ alkyl (oligo- or poly-oxa C₁₋₃ alkylene) is oligo- or polyglycerol ethers.

7. A pharmaceutical composition according to any of claims 1 to 6 wherein the polysaccharide has 1,4-linked monosaccharide units.

8. A pharmaceutical composition according to claim 7 wherein each non-ionic hydrophilic group is substituted at the C6 position of a monosaccharide unit.

9. A pharmaceutical composition according to claim 7 or claim 8 in which the hydrophobic group is substituted at the C2 position.

10. A pharmaceutical composition according to any preceding claim wherein the degree of substitution by non-ionic hydrophilic groups is 0.1-1.5 per monosaccharide unit.

11. A pharmaceutical composition according to claim 10 wherein the degree of substitution by non-ionic hydrophilic groups is at least 0.9 per monosaccharide unit.

12. A pharmaceutical composition according to any preceding claim wherein the ratio of hydrophilic: hydrophobic groups is in the range 100:1 to 1:2.

13. A pharmaceutical composition according to claim 12 wherein the ratio of hydrophilic: hydrophobic groups is in the range 10:1 to 2:1.

14. A pharmaceutical composition according to claim 13 wherein the ratio of hydrophilic: hydrophobic groups is in the range 5:1 to 2:1.

15. A pharmaceutical composition according to any preceding claim wherein the hydrophobic group is joined to a monosaccharide unit by an amide, ester, ether or amine linkage.

16. A pharmaceutical composition according to any preceding claim wherein the polysaccharide is a derivative of chitosan, pullulan or dextran.

17. A pharmaceutical composition according to any preceding claim which is an N-substituted derivative of a poly-amino glycan.

18. A pharmaceutical composition according to claim 17 wherein said N-substituted derivative of a poly-amino glycan is an N-acyl glycol chitosan.

19. A pharmaceutical composition according to claim 17 or claim 18 wherein said N-acyl glycol chitosan is N-palmitoyl glycol chitosan.

20. A composition comprising particles formed from a compound having the formula: 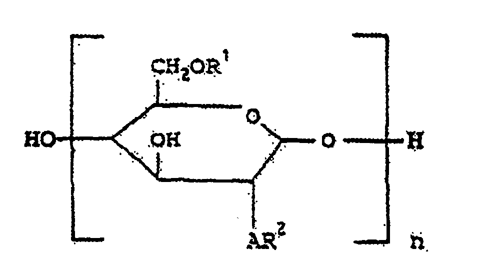 wherein each R' is selected from hydrogen, mono-and oligo-hydroxy C₁₋₆ alkyl, mono-and oligo-hydroxy substituted C₂₋₆ acyl, C₁₋₂ alkoxy alkyl, oligo- or poly-(oxa C₁₋₃ alkylene) and C₁₋₄ alkyl (oligo- or poly-oxa C₁₋₃ alkylene), provided that at least one of the groups R¹ is other than hydrogen;
A is -NH- or -O-;
R² is selected from hydrogen, C₁₂₋₂₄ alkyl, -alkanoyl, -alkenyl -alkenoyl, -alkynyl or alkynoyl, provided that at least one of the groups R² is other than hydrogen; and n is 5-2000.

21. A composition according to claim 20 wherein said C₁₋₂ alkoxy alkyl has one or more hydroxy groups substituted on the alkoxy or alkylene groups.

22. A composition according to claim 20 wherein said oligo- or poly- (oxa C₁₋₃ alkylene) is polyoxyethylene comprising up to 120 ethylene oxide units.

23. A composition according to claim 20 wherein said C₁₋₄ alkyl (oligo- or poly-oxa C₁₋₃ alkylene) is hydroxy substituted.

24. A composition according to claim 23 wherein said C₁₋₄ alkyl (oligo- or poly-oxa C₁₋₃ alkylene) is hydroxy substituted using polyglycerol ethers.

25. A composition according to claim 24 wherein said C₁₋₄ alkyl (oligo- or poly-oxa C₁₋₃ alkylene) is hydroxy substituted using polyglycerol ethers containing up to 10 glycerol units.

26. A composition according to any of claims 20 to 25 wherein R¹ is -CH₂CH₂OH or -CH₂CH(OH)CH₂OH.

27. A composition according to any of claims 20 to 26 wherein R² is C₁₆₋₁₈ acyl.

28. A composition according to any of claims 20 to 27 wherein A is -NH-.

29. A composition or a pharmaceutical composition according to any preceding claim which comprises an aqueous vehicle in which the particles are suspended.

30. A composition or a pharmaceutical composition according to any preceding claim which additionally comprises cholesterol or a derivative thereof.

31. A composition or a pharmaceutical composition according to claim 30 which further comprises a steric stabilizer.

32. A composition or a pharmaceutical composition according to claim 31 wherein said steric stabilizer is a non-ionic amphiphilic compound.

33. A composition or a pharmaceutical composition according to claim 32 wherein said steric stabilizer is a poly-24-oxyethylene cholesteryl ether.

34. A composition or a pharmaceutical composition according to any preceding claim further comprising a pharmacologically acceptable carrier.

35. A composition or pharmaceutical composition according to any preceding claim which comprises a pharmaceutically active ingredient associated with the particles.

36. A composition or pharmaceutical composition according to claim 35 which comprises an entrapped pharmaceutically active ingredient

37. A composition or pharmaceutical composition according to either of claims 35 and 36 which comprises a covalently conjugated pharmaceutically active ingredient.

38. A composition or pharmaceutical composition according to any of claims 35 to 37 in which the pharmaceutically active ingredient is a peptide or protein therapeutic compound or DNA.

39. A composition or a pharmaceutical composition according to claim 38 wherein said DNA is a gene for gene therapy or gene vaccination.

40. A composition or pharmaceutical composition according to any preceding claim for use in therapy.

41. Use of a composition or pharmaceutical composition according to any preceding claim for the manufacture of a medicament for use in therapy.

42. Use of a composition or pharmaceutical composition according to any preceding claim and a pharmaceutically active ingredient for the manufacture of a medicament for use in therapy.

43. A polysaccharide derivative bearing at least one non-ionic hydrophilic group and at least one hydrophobic group per polysaccharide molecule, wherein said hydrophilic group is joined at the C6 position of a monosaccharide unit of said polysaccharide molecule, and said hydrophobic group is joined at the C2 position of a monosaccharide unit of said polysaccharide molecule via an amide linkage or amine linkage, and wherein said hydrophobic group comprises a C₁₂₋₂₄ alkyl, alkenyl, alkynyl or acyl residue.

44. A polysaccharide derivative bearing at least one non-ionic hydrophilic group and at least one hydrophobic group per polysaccharide molecule for use in therapy, wherein said hydrophilic group is attached to a monosaccharide unit of said polysaccharide molecule and said hydrophobic group is attached to a monosaccharide unit of said polysaccharide molecule, and wherein said hydrophobic group comprises a C₁₂₋₂₄ alkyl, alkenyl, alkynyl or acyl residue.

45. Use of a polysaccharide derivative bearing at least one non-ionic hydrophilic group and at least one hydrophobic group per polysaccharide molecule for the manufacture of a medicament for use in therapy, wherein said hydrophilic group is attached to a monosaccharide unit of said polysaccharide molecule and said hydrophobic group is attached to a monosaccharide unit of said polysaccharide molecule and wherein said said hydrophobic group comprises a C₁₂₋₂₄ alkyl, alkenyl, alkynyl or acyl residue.

## Patentansprüche

1. Pharmazeutische Zusammensetzung, aufweisend Partikel, welche Partikel unverzweigte Polysaccharid-Derivate aufweisen, wobei jedes Polysaccharid-Derivat mindestens eine nichtionische hydrophile Gruppe trägt und mindestens eine hydrophobe Gruppe pro Polysaccharid-Molekül, und einen pharmazeutisch zulässigen Träger, wobei die hydrophile Gruppe an einer Monosaccharid-Einheit des Polysaccharid-Moleküls angebracht ist und die hydrophobe Gruppe an einer Monosaccharid-Einheit des Polysaccharid-Moleküls angebracht ist und wobei die hydrophobe Gruppe einen C₁₂₋₂₄-Alkyl-, -Alkenyl-, -Alkinyl- oder -Acyl-Rest aufweist.

2. Pharmazeutische Zusammensetzung nach Anspruch 1, worin die nichtionische hydrophile Gruppe eine Gruppe R¹ ist, worin R¹ ausgewählt ist aus Mono- und Oligo-Hydroxy-C₁₋₆-Alkyl, Mono- und Oligo-Hydroxy-substituiertes C₂₋₆-Acyl, C₁₋₂-Alkoxyalkyl, Oligo- oder Poly-(Oxa-C₁₋₃-alkylen) und C₁₋₄-Alkyl(Oligo- oder Polyoxa-C₁₋₃-alkylen); und worin R¹ über eine Etherverknüpfung mit einer Monosaccharid-Einheit des Polysaccharids verbunden ist.

3. Pharmazeutische Zusammensetzung nach Anspruch 2, bei welcher das C₁₋₂-Alkoxyalkyl eine oder mehrere Gruppen hat, substituiert an den Alkoxy- oder Alkylen-Gruppen.

4. Pharmazeutische Zusammensetzung nach Anspruch 2, worin das Oligo- oder Poly-(Oxa-C₁₋₃-alkylen) Polyoxyethylen ist, das bis zu 120 Ethylenoxid-Einheiten aufweist.

5. Pharmazeutische Zusammensetzung nach Anspruch 2, bei welcher das C₁₋₄-Alkyl(oligo- oder polyoxa-C₁₋₃-alkylen) Hydroxy-substituiert ist.

6. Pharmazeutische Zusammensetzung nach Anspruch 5, worin das Hydroxysubstituierte C₁₋₄-Alkyl(Oligo- oder Polyoxa-C₁₋₃-alkylen) Oligo- oder Polyglycerinether ist.

7. Pharmazeutische Zusammensetzung nach einem der Ansprüche 1 bis 6, worin das Polysaccharid 1,4-verknüpfte Monosaccharid-Einheiten hat.

8. Pharmazeutische Zusammensetzung nach Anspruch 7, worin jede nichtionische hydrophile Gruppe substituiert ist an der C₆-Position einer Monosaccharid-Einheit.

9. Pharmazeutische Zusammensetzung nach Anspruch 7 oder Anspruch 8, worin die hydrophobe Gruppe an der C₂-Position substituiert ist.

10. Pharmazeutische Zusammensetzung nach einem der vorgenannten Ansprüche, worin der Substitutionsgrad durch nichtionische hydrophile Gruppen 0,1 bis 1,5 pro Monosaccharid-Einheit beträgt.

11. Pharmazeutische Zusammensetzung nach Anspruch 10, worin der Substitutionsgrad durch nichionische hydrophile Gruppen mindestens 0,9 pro Monosaccharid-Einheit beträgt.

12. Pharmazeutische Zusammensetzung nach einem der vorgenannten Ansprüche, worin das Verhältnis von hydrophilen:hydrophoben Gruppen im Bereich von 100:1 bis 1:2 liegt.

13. Pharmazeutische Zusammensetzung nach Anspruch 12, worin das Verhältnis von hydrophilen:hydrophoben Gruppen im Bereich von 10:1 bis 2:1 liegt.

14. Pharmazeutische Zusammensetzung nach Anspruch 13, worin das Verhältnis von hydrophilen:hydrophoben Gruppen im Bereich von 5:1 bis 2:1 liegt.

15. Pharmazeutische Zusammensetzung nach einem der vorgenannten Ansprüche, worin die hydrophobe Gruppe über eine Amid-, Ester-, Ether, oder Amin-Verknüpfung mit einer Monosaccharid-Einheit verbunden ist.

16. Pharmazeutische Zusammensetzung nach einem der vorgenannten Ansprüche, worin das Polysaccharid ein Derivat von Chitosan, Pullulan oder Dextran ist.

17. Pharmazeutische Zusammensetzung nach einem der vorgenannten Ansprüche, die ein N-substituiertes Derivat von Polyaminoglykan ist.

18. Pharmazeutische Zusammensetzung nach Anspruch 17, worin das N-substituicrte Derivat eines Polyaminoglykans ein N-Acylglykolchitosan ist.

19. Pharmazeutische Zusammensetzung nach Anspruch 17 oder 18, worin das N-Acylglykolchitosan N-Palmitoylglykolchitosan ist.

20. Zusammensetzung, aufweisend Partikel, die erzeugt sind aus einer Verbindung mit der Formel: 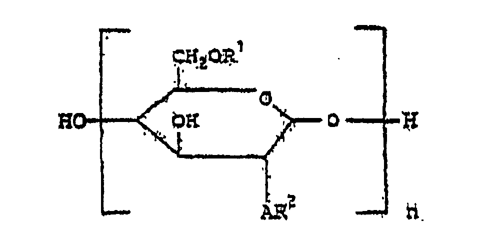 worin jedes R¹ ausgewählt ist aus: Wasserstoff, Mono- und Oligo-Hydroxy-C₁₋₆-Alkyl, Mono- und Oligo-Hydroxy-substituiert-C₂₋₆-Acyl, C₁₋₂-Alkoxyalkyl, Oligo- oder Poly-(oxa-C₁₋₃-alkylen) und C₁₋₄-Alkyl(Oligo- oder Polyoxa-C₁₋₃-alkylen) unter der Voraussetzung, dass mindestens eine der Gruppen R¹ nicht Wasserstoff ist;
A ist -NH- oder -O-;
R² ist ausgewählt aus Wasserstoff, C₁₂₋₂₄-Alkyl, -Alkanoyl, -Alkenyl, -Alkenoyl, -Alkinyl oder -Alkinoyl unter der Voraussetzung, dass mindestens eine der Gruppen R² nicht Wasserstoff ist; und
n beträgt 5 bis 2.000.

21. Zusammensetzung nach Anspruch 20, worin das C₁₋₂-Alkoxyalkyl eine oder mehrere Hydroxy-Gruppen substituiert an den Alkoxy- oder Alkylen-Gruppen hat.

22. Zusammensetzung nach Anspruch 20, worin das Oligo- oder Poly-(oxa-C₁₋₃alkylen) Polyoxyethylen ist, das bis zu 120 Ethylenoxid-Einheiten aufweist.

23. Zusammensetzung nach Anspruch 20, worin das C₁₋₄-Alkyl(Oligo- oder Polyoxa-C₁₋₃-alkylen) Hydroxy-substituiert ist.

24. Zusammensetzung nach Anspruch 23, worin das C₁₋₄-Alkyl(Oligo- oder Polyoxa-C₁₋₃-alkylen) Hydroxy-substituiert ist unter Verwendung von Polyglycerinethern.

25. Zusammensetzung nach Anspruch 24, worin das C₁₋₄-Alkyl(Oligo- oder Polyoxa-C₁₋₃-alkylen) Hydroxy-substituiert ist unter Anwendung von Polyglycerinethern, die bis zu 10 Glycerin-Einheiten enthalten.

26. Zusammensetzung nach einem der Ansprüche 20 bis 25, worin R¹ -CH₂CH₂OH oder -CH₂CH(OH)CH₂OH ist.

27. Zusammensetzung nach einem der Ansprüche 20 bis 26, worin R² C₁₆₋₁₈-Acyl ist.

28. Zusammensetzung nach einem der Ansprüche 20 bis 27, worin A -NH- ist.

29. Zusammensetzung oder pharmazeutische Zusammensetzung nach einem der vorgenannten Ansprüche, die ein wässriges Vehikel aufweist, worin die Partikel suspendiert sind.

30. Zusammensetzung oder pharmazeutische Zusammensetzung nach einem der vorgenannten Ansprüche, die zusätzlich Cholesterin oder ein Derivat davon aufweist.

31. Zusammensetzung oder pharmazeutische Zusammensetzung nach Anspruch 30, die ferner ein sterisches Stabilisiermittel aufweist.

32. Zusammensetzung oder pharmazeutische Zusammensetzung nach Anspruch 31, worin das sterische Stabilisiermittel eine nichtionische amphiphile Verbindung ist.

33. Zusammensetzung oder pharmazeutische Zusammensetzung nach Anspruch 32, worin das sterische Stabilisiermittel ein Poly-24-oxyethylencholesterylether ist.

34. Zusammensetzung oder pharmazeutische Zusammensetzung nach einem der vorgenannten Ansprüche, ferner aufweisend einen pharmalcologisch zulässigen Träger.

35. Zusammensetzung oder pharmazeutische Zusammensetzung nach einem der vorgenannten Ansprüche, die einen pharmazeutisch aktiven Wirkstoff in Verbindung mit den Partikeln aufweist.

36. Zusammensetzung oder pharmazeutische Zusammensetzung nach Anspruch 35, die einen eingeschlossenen pharmazeutisch aktiven Wirkstoff aufweist.

37. Zusammensetzung oder pharmazeutische Zusammensetzung nach einem der Ansprüche 35 und 36, die einen kovalent konjugierten pharmazeutisch aktiven Wirkstoff aufweist.

38. Zusammensetzung oder pharmazeutische Zusammensetzung nach einem der Ansprüche 35 bis 37, worin der pharmazeutisch aktive Wirkstoff eine Peptid- oder Protein-therapeutische Verbindung oder DNA ist.

39. Zusammensetzung oder pharmazeutische Zusammensetzung nach Anspruch 38, worin die DNA ein Gen für die Gentherapie oder Genvakzination ist.

40. Zusammensetzung oder pharmazeutische Zusammensetzung nach einem der vorgenannten Ansprüche zur Verwendung in der Therapie.

41. Verwendung einer Zusammensetzung oder pharmazeutischen Zusammensetzung nach einem der vorgenannten Ansprüche für die Herstellung eines Medikaments zur Verwendung in der Therapie.

42. Verwendung einer Zusammensetzung oder pharmazeutischen Zusammensetzung nach einem der vorgenannten Ansprüche und eines pharmazeutisch aktiven Wirkstoffes für die Herstellung eines Medikaments zur Verwendung in der Therapie.

43. Polysaccharid-Derivat, mindestens eine nichtionische hydrophile Gruppe tragend und mindestens eine hydrophobe Gruppe pro Polysaccharid-Molekül, worin die hydrophile Gruppe in der C₆-Position mit einer Monosaccharid-Einheit des Polysaccharid-Moleküls verbunden ist und worin die hydrophobe Gruppe in der C₂-Position mit einer Monosaccharid-Einheit des Polysaccharid-Moleküls über eine Amid-Verknüpfung oder Amin-Verknüpfung verbunden ist und worin die hydrophobe Gruppe einen C₁₂₋₂₄-Alkyl-, -Alkenyl-, -Alkinyl- oder -Acyl-Rest aufweist.

44. Polysaccharid-Derivat, mindestens eine nichtionische hydrophile Gruppe tragend und mindestens eine hydrophobe Gruppe pro Polysaccharid-Molekül zur Verwendung in der Therapie, worin die hydrophile Gruppe an einer Monosaccharid-Einheit des Polysaccharid-Moleküls angebracht ist und worin die hydrophobe Gruppe an einer Monosaccharid-Einheit des Polysaccharid-Moleküls angebracht ist und worin die hydrophobe Gruppe einen C₁₂₋₂₄-Alkyl-, -Alkenyl-, -Alkinyl- oder -Acyl-Rest aufweist.

45. Verwendung eines Polysaccharid-Derivats, mindestens eine nichtionische hydrophile Gruppe tragend und mindestens eine hydrophobe Gruppe pro Polysaccharid-Molekül für die Herstellung eines Medikaments zur Verwendung in der Therapie, worin die hydrophile Gruppe an einer Monosaccharid-Einheit des Polysaccharid-Moleküls angebracht ist und worin die hydrophobe Gruppe an einer Monosaccharid-Einheit des Polysaccharid-Moleküls angebracht ist und worin die hydrophobe Gruppe einen C₁₂₋₂₄-Alkyl-, -Alkenyl-, -Alkinyl- oder -Acyl-Rest aufweist.

## Revendications

1. Une composition pharmaceutique comprenant des particules, lesdites particules comportant des dérivés polysaccharidiques linéaires portant chacun au moins un groupement hydrophile non ionique et au moins un groupement hydrophobe par molécule polysaccharidique et un véhicule pharmaceutiquement acceptable, dans laquelle ledit groupement hydrophile est lié à une unité monosaccharidique de ladite molécule polysaccharidique et ledit groupement hydrophobe est lié à une unité monosaccharidique de ladite molécule polysaccharidique et ledit groupement hydrophobe comprend un résidu alkyle, alkényle, alkynyle ou acyle en C₁₂₋₂₄.

2. Une composition pharmaceutique selon la revendication 1, dans laquelle le groupement hydrophile non ionique est un groupement R¹, où R¹ est sélectionné parmi le groupe consistant en un alkyle mono- ou oligohydroxy en C₁₋₆, un acyle mono- ou oligohydroxy substitué en C₂₋₆, un alkoxyalkyle en C₁₋₂, un oligo- ou poly-[(oxa)alkylène] en C₁₋₃ et un alkyl en C₁₋₄-[oligo- ou poly- (oxa)alkylène)] en C₁₋₃ ; et dans laquelle le groupement R¹ est lié par un pont éther à une unité monosaccharidique du polysaccharide.

3. Une composition pharmaceutique selon la revendication 2, dans laquelle ledit groupement alkoxyalkyle en C₁₋₂ est substitué par un ou plusieurs radicaux hydroxy sur les groupements alkoxy ou alkylène.

4. Une composition pharmaceutique selon la revendication 2, dans laquelle ledit groupement oligo- ou poly-[(oxa)alkylène en C₁₋₃] est un polyoxyéthylène qui comporte jusqu'à 120 unités d'oxyde d'éthylène.

5. Une composition pharmaceutique selon la revendication 2, dans laquelle ledit groupement alkyl en C₁₋₄-[oligo- ou poly- (oxa)alkylène)] en C₁₋₃ est substitué par un radical hydroxy.

6. Une composition pharmaceutique selon la revendication 5, dans laquelle ledit groupement alkyl en C₁₋₄-[oligo- ou poly- (oxa)alkylène)] en C₁₋₃ substitué par un radical hydroxy est un éther d'oligo- ou de polyglycérol.

7. Une composition pharmaceutique selon l'une quelconque des revendications 1 à 6, dans laquelle le polysaccharide présente des unités monosaccharidiques à liaisons 1, 4.

8. Une composition pharmaceutique selon la revendication 7, dans laquelle le groupement hydrophile non ionique est un substituant à la position C6 d'une unité monosaccharidique.

9. Une composition pharmaceutique selon la revendication 7 ou la revendication 8, dans laquelle le groupement hydrophobe est un substituant à la position C2.

10. Une composition pharmaceutique selon l'une quelconque des revendications précédentes, dans laquelle le degré de substitution par des groupements hydrophiles non ioniques est de 0,1-1,5 par unité monosaccharidique.

11. Une composition pharmaceutique selon la revendication 10, dans laquelle le degré de substitution par des groupements hydrophiles non ioniques est d'au moins 0,9 par unité monosaccharidique.

12. Une composition pharmaceutique selon l'une quelconque des revendications précédentes, dans laquelle le rapport entre les groupements hydrophiles:hydrophobes est compris entre 100:1 et 1:2.

13. Une composition pharmaceutique selon la revendication 12, dans laquelle le rapport entre les groupements hydrophiles:hydrophobes est compris entre 10:1 et 2:1.

14. Une composition pharmaceutique selon la revendication 13, dans laquelle le rapport entre les groupements hydrophiles:hydrophobes est compris entre 5:1 et 2:1.

15. Une composition pharmaceutique selon l'une quelconque des revendications précédentes, dans laquelle le groupement hydrophobe est lié à une unité monosaccharidique par un pont amide, ester, éther ou amine.

16. Une composition pharmaceutique selon l'une quelconque des revendications précédentes, dans laquelle le polysaccharide est un dérivé du chitosane, du pullulane ou du dextranc.

17. Une composition pharmaceutique selon l'une quelconque des revendications précédentes, qui correspond à un dérivé N-substitué d'un polyamimoglycane.

18. Une composition pharmaceutique selon la revendication 17, dans laquelle le dérivé N-substitué d'un polyaminoglycane est un N-acylglycolchitosane.

19. Une composition pharmaceutique selon la revendication 17 ou 18, dans laquelle le N-acylglycolchitosane est un N-palmitoylglycolchitosane

20. Une composition pharmaceutique comprenant des particules formées à partir d'un composé de la formule: 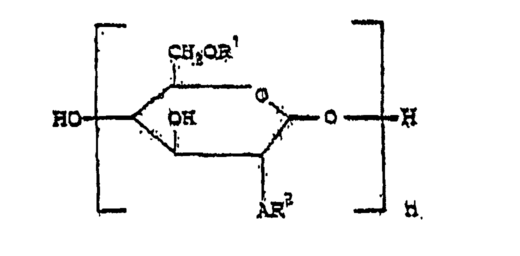 dans laquelle chaque groupement R¹ est sélectionné parmi le groupe consistant en un atome d'hydrogène, un alkyle mono- ou oligohydroxy en C₁₋₆, un acyle mono- ou oligohydroxy substitué en C₂₋₆, un alkoxyalkyle en C₁₋₂, un oligo- ou poly-[(oxa)alkylène] en C₁₋₃ et un alkyl en C₁₋₄-[oligo- ou poly- (oxa)alkylène)] en C₁₋₃, à condition que un au moins des groupements R¹ ne représente pas un atome d'hydrogène;
A représente -NH- ou -O-;
R² est sélectionné parmi le groupe consistant en un atome d'hydrogène ou un groupement alkyle, alkanoyle, alkényle, alkénoyle, alkynyle ou alkynoyle en C₁₂₋₂₄, à condition que un au moins des groupement R² ne représente pas un atome d'hydrogène ; et n est compris entre 5 et 2 000.

21. Une composition selon la revendication 20, dans laquelle ledit groupement alkoxyalkyle en C₁₋₂ est substitué par un ou plusieurs radicaux hydroxy sur les groupements alkoxy ou alkylène.

22. Une composition selon la revendication 20, dans laquelle ledit groupement oligo-ou poly-[(oxa)alkylène en C₁₋₃] est un polyoxyéthylène qui comporte jusqu'à 120 unités d'oxyde d'éthylène.

23. Une composition selon la revendication 20, dans laquelle ledit groupement alkyl en C₁₋₄-[oligo- ou poly- (oxa)alkylène)] en C₁₋₃ est substitué par un radical hydroxy.

24. Une composition selon la revendication 23, dans laquelle ledit groupement alkyl en C₁₋₄-[oligo- ou poly- (oxa)alkylène)] en C₁₋₃ est substitué par un radical hydroxy en utilisant des éthers de polyglycérol.

25. Une composition selon la revendication 24, dans laquelle ledit groupement alkyl en C₁₋₄-[oligo- ou poly- (oxa)alkylène)] en C₁₋₃ est substitué par un radical hydroxy en utilisant des éthers de polyglycérol contenant jusqu'à 10 unités glycérol.

26. Une composition selon l'une quelconque des revendications 20 à 25, dans laquelle R¹ représente -CH₂CH₂OH- ou -CH₂CH(OH)CH₂OH.

27. Une composition selon l'une quelconque des revendications 20 à 26, dans laquelle R² représente un groupement acyle en C₁₆₋₁₈.

28. Une composition selon l'une quelconque des revendications 20 à 27, dans laquelle A représente -NH-.

29. Une composition ou une composition pharmaceutique selon l'une quelconque des revendications précédentes, qui comprend un véhicule aqueux dans lequel les particules sont en suspension.

30. Une composition ou une composition pharmaceutique selon l'une quelconque des revendications précédentes, qui comprend également du cholestérol ou un dérivé de celui-ci.

31. Une composition ou une composition pharmaceutique selon la revendication 30, qui comprend également un stabilisateur stérique.

32. Une composition ou une composition pharmaceutique selon la revendication 31, dans laquelle ledit stabilisateur stérique est un composé amphiphile non ionique.

33. Une composition ou une composition pharmaceutique selon la revendication 32, dans laquelle ledit stabilisateur stérique est un éther cholestérylique de poly-24-oxyéthylène.

34. Une composition ou une composition pharmaceutique selon l'une quelconque des revendications précédentes, qui comprend également un véhicule pharmaceutiquement acceptable.

35. Une composition ou une composition pharmaceutique selon l'une quelconque des revendications précédentes, qui comprend un principe pharmaceutiquement actif associé aux dites particules.

36. Une composition ou une composition pharmaceutique selon la revendication 35, qui comprend un principe pharmaceutiquement actif piégé.

37. Une composition ou une composition pharmaceutique selon la revendication soit 35, soit 36, qui comprend un principe pharmaceutiquement actif conjugué par une liaison covalente.

38. Une composition ou une composition pharmaceutique selon l'une quelconque des revendications 35 à 37, dans laquelle le principe pharmaceutiquement actif est un peptide, un composé protéique thérapeutique ou un fragment d'ADN.

39. Une composition ou une composition pharmaceutique selon la revendication 38, dans laquelle ledit fragment d'ADN est un gène destiné à une thérapie génétique ou une vaccination génétique.

40. Une composition ou une composition pharmaceutique selon l'une quelconque des revendications précédentes, destinée à un usage thérapeutique.

41. L'utilisation d'une composition ou d'une composition pharmaceutique selon l'une quelconque des revendications précédentes dans la fabrication d'un médicament à visée thérapeutique.

42. L'utilisation d'une composition ou d'une composition pharmaceutique selon l'une quelconque des revendications précédentes et d'un principe pharmaceutiquement actif dans la fabrication d'un médicament à visée thérapeutique.

43. Un dérivé polysaccharidique portant au moins un groupement hydrophile non ionique et au moins un groupement hydrophobe par molécule polysaccharidique, dans lequel ledit groupement hydrophile est lié en position C6 à une unité monosaccharidique de ladite molécule polysaccharidique, et ledit groupement hydrophobe est lié en position C2 à une unité monosaccharidique de ladite molécule polysaccharidique par un pont amide ou par un pont amine, et dans lequel ledit groupement hydrophobe comprend un résidu alkyle, alkényle, alkynyle ou acyle en C₁₂₋₂₄.

44. Un dérivé polysaccharidique portant au moins un groupement hydrophile non ionique et au moins un groupement hydrophobe par molécule polysaccharidique et destiné à un usage thérapeutique, dans lequel ledit groupement hydrophile est lié à une unité monosaccharidique de ladite molécule polysaccharidique et ledit groupement hydrophobe est lié à une unité monosaccharidique de ladite molécule polysaccharidique, et dans lequel ledit groupement hydrophobe comprend un résidu alkyle, alkényle, alkynyle ou acyle en C₁₂₋₂₄.

45. L'utilisation d'un dérivé polysaccharidique portant au moins un groupement hydrophile non ionique et au moins un groupement hydrophobe par molécule polysaccharidique dans la fabrication d'un médicament à visée thérapeutique, dans lequel ledit groupement hydrophile est lié à une unité monosaccharidique de ladite molécule polysaccharidique et ledit groupement hydrophobe est lié à une unité monosaccharidique de ladite molécule polysaccharidique, et dans lequel ledit groupement hydrophobe comprend un résidu alkyle, alkényle, alkynyle ou acyle en C₁₂₋₂₄.
